# EUROPEAN PATENT SPECIFICATION

(11) **EP 2 625 288 B1**
(45) Date of publication and mention of the grant of the patent: **25.05.2016**
(21) Application number: 11770415.5
(22) Date of filing: 10.10.2011
(51) Int. Cl.: C12Q 1/68

(54) **METHOD FOR DETERMINING CANCER PATIENT SURVIVAL BASED ON ANALYZING TUMOR-INFILTRATING OVERALL T-LYMPHOCYTES**
VERFAHREN ZUR BESTIMMUNG DES ÜBERLEBENS EINES KREBSPATIENTEN AUF BASIS DER ANALYSE DER TUMORINFILTRATION INSGESAMT
MÉTHODE DE DÉTERMINATION DU PRONOSTIC DE SURVIE D'UN PATIENT ATTEINT DE CANCER BASÉE SUR L'ANALYSE DES LYMPHOCYTES T GLOBAUX INFILTRANT LA TUMEUR

(30) Priority: 08.10.2010 EP 10187046
(43) Date of publication of application: 14.08.2013
(73) Proprietor: Epiontis GmbH, 12489 Berlin (DE)
(72) Inventor: OLEK, Sven, 12207 Berlin (DE); SCHWACHULA, Tim, 12437 Berlin (DE); BARON, Udo, 10245 Berlin (DE)
(74) Representative: Krauss, Jan
(86) International application number: PCT/EP2011/067660
(87) International publication number: WO 2012/045888

(56) References cited:
- EP-A1- 1 777 523
- EP-A1- 2 141 245
- EP-A1- 2 199 411
- BLAISE CLARKE ET AL: "Intraepithelial T cells and prognosis in ovarian carcinoma: novel associations with stage, tumor type, and BRCA1 loss", MODERN PATHOLOGY, vol. 22, no. 3, 1 March 2009 (2009-03-01), pages 393-402, XP55013266, ISSN: 0893-3952, DOI: 10.1038/modpathol.2008.191
- ZHANG LIN ET AL: "Intratumoral T cells, recurrence, and survival in epithelial ovarian cancer", NEW ENGLAND JOURNAL OF MEDICINE, THE, MASSACHUSETTS MEDICAL SOCIETY, WALTHAM, MA, US, vol. 348, no. 3, 16 January 2003 (2003-01-16), pages 203-213, XP002390426, ISSN: 0028-4793, DOI: 10.1056/NEJMOA020177
- BARON UDO ET AL: "DNA demethylation in the human FOXP3 locus discriminates regulatory T cells from activated FOXP3(+) conventional T cells", EUROPEAN JOURNAL OF IMMUNOLOGY, WILEY - V C H VERLAG GMBH & CO. KGAA, DE, vol. 37, no. 9, 1 September 2007 (2007-09-01), pages 2378-2389, XP002503237, ISSN: 0014-2980, DOI: 10.1002/EJI.200737594
- DATABASE MEDLINE [Online] US NATIONAL LIBRARY OF MEDICINE (NLM), BETHESDA, MD, US February 2011 SEHOULI JALID ET AL: 'Epigenetic quantification of tumor-infiltrating T-lymphocytes.' Database accession no. NLM20962591 & SEHOULI JALID ET AL: "Epigenetic quantification of tumor-infiltrating T-lymphocytes.", EPIGENETICS : OFFICIAL JOURNAL OF THE DNA METHYLATION SOCIETY FEB 2011, vol. 6, no. 2, February 2011 (2011-02), pages 236-246, ISSN: 1559-2308

## Description

The present invention relates to a method, in vitro, for determining cancer patient survival, comprising analyzing the number and/or amount of tumor-infiltrating overall T-lymphocytes (oTLs) based on the methylation status of at least one CpG position in one or more of the genes for CD3 γ, -δ, and -ε in a tumor sample derived from said cancer patient, wherein a high number and/or amount of oTLs is indicative for a better survival of said cancer patient in a non-breast cancer, wherein in breast cancer a high number and/or amount of oTLs is indicative for a inferior survival of said patient. The present invention also relates to the use of a respective kit in the methods of the invention.

### Background of the invention

Establishment of malignant tumors depends on favorable growth kinetics of tumor cells and tumor strategies to escape from immune surveillance. Until recently, therapeutic anti-tumor strategies focused on eradication of malignant cells, which on its own is mostly unable to cure later stage disease. The immune system was recognized as additional target, due to its ambiguous role in exhibiting host protection on one hand, and facilitating tumor growth on the other hand.

Antigen-specific host protection is achieved via the adaptive immune system, consisting of Band T-lymphocytes. The latter are collectively defined by expression of the T cell receptor (TCR) complex including the T-cell surface glycoprotein CD3. Tumor cells express specific antigens, thus becoming targets for T cell-mediated immune responses. Mouse experiments confirm a role of T-cells in cancer immunosurveillance and tumor infiltrating overall T-lymphocytes (oTL) inhibit tumor growth in colorectal and ovarian cancers. Also, increased CD3 mRNA expression levels in the tumor correlate with improved outcome, preventing tumor recurrence in colorectal cancer (Galon, J., et al., Type, density, and location of immune cells within human colorectal tumors predict clinical outcome. Science, 2006. 313(5795): p. 1960-4).

The function of regulatory T cells (Treg) is converse to the role of effector T-lymphocytes. They control effector T-cell responses and mediate tolerance. Immunological tolerance refers to the ability of the adaptive immune system to spare certain antigens from immunological attack, counteracting excessive immune reactions. Treg are commonly identified by the expression of CD3, CD4, CD25, and the transcription factor FOXP3. An array of further markers including CD127, CTLA4, GITR, CD45RA or CD103 is used for Treg isolation and characterization in order to increase specificity.

Even though almost all cells in an individual contain the exact same complement of DNA code, higher organisms must impose and maintain different patterns of gene expression in the various types of tissue. Most gene regulation is transitory, depending on the current state of the cell and changes in external stimuli. Persistent regulation, on the other hand, is a primary role of epigenetics - heritable regulatory patterns that do not alter the basic genetic coding of the DNA. DNA methylation is the archetypical form of epigenetic regulation; it serves as the stable memory for cells and performs a crucial role in maintaining the long-term identity of various cell types.

The primary target of methylation is the two-nucleotide sequence Cytosine-Guanine (a'CpG site'); within this context cytosine (C) can undergo a simple chemical modification to become 5-methyl-cytosine. In the human genome, the CG sequence is much rarer than expected, except in certain relatively dense clusters called 'CpG islands'. CpG islands are frequently associated with gene promoters, and it has been estimated that more than half of the human genes have CpG islands (Antequera and Bird, Proc Natl Acad Sci USA 90: 11995-9, 1993).

Aberrant methylation of DNA is frequently associated with the transformation from healthy to cancerous cells. Among the observed effects are genome-wide hypomethylation, increased methylation of tumor suppressor genes, and hypomethylation of many oncogenes (reviewed, for example, by Jones and Laird, Nature Genetics 21:163-167, 1999; Esteller, Oncogene 21:5427-5440, 2002; and Laird, Nature Reviews/Cancer 3:253-266, 2003). Methylation profiles have been recognized to be tumor specific (i.e., changes in the methylation pattern of particular genes or even individual CpGs are diagnostic of particular tumor types), and there is now an extensive collection of diagnostic markers for bladder, breast, colon, esophagus, stomach, liver, lung, and prostate cancers (summarized, for example, by Laird, Nature Reviews/Cancer 3:253-266, 2003).

Hamerman et al. (in: Hamerman JA, Page ST, Pullen AM. Distinct methylation states of the CD8 beta gene in peripheral T cells and intraepithelial lymphocytes. J Immunol. 1997 Aug 1;159(3):1240-6) distinguish between CD4 and CD8 T-lymphocytes.

EP 1 213 360 describes a method of identifying a cell, tissue or nucleus, comprising collecting information on the methylation pattern of DNA isolated from the cell, tissue or nucleus and analyzing the resultant information.

WO 2004/050706 describes a sub-group of T-cells, and relates to characteristics of regulatory T-cells which define them as such. The application also describes the uses of such T-cells, compositions comprising them, and chemokines which recruit them in the modulation of an immune response.

EP 1 826 279 describes a method, in particular an *in vitro* method, for identifying FoxP3-positive regulatory T cells, preferably CD25⁺ CD4⁺ regulatory T cells of a mammal, comprising analyzing the methylation status of at least one CpG position in the gene foxp3 or an orthologous or paralogous gene thereof, and the use of DNA-methylation analysis of the gene of the transcription factor FoxP3 for a detection and quality assurance and control of regulatory T cells.

Finally, EP 2 199 411 describes a method for identifying CD3CD4 and/or CD3CD8 positive T lymphocytes of a mammal, wherein said method comprises analyzing the methylation status of at least one CpG position in the CD35/γ/ε genes, in particular their "upstream" regulatory regions, and in particular the promoter and other conserved regions of the gene for CD3, wherein a demethylation of at least one CpG in the analyzed sample to at least 90% is indicative for memory and naive CD4⁺ T lymphocytes and memory and naive CD8⁺ T lymphocytes.

The lack of a more detailed understanding of the clinical role of T lymphocytes, such as tumor infiltrating overall T-lymphocytes (oTL), largely results from technical difficulties associated with mRNA analysis, immunohistochemistry (IHC) or flow cytometry in the analysis of tissue-infiltrating immune cells. All three technologies are afflicted with limitations when applied in a quantitative manner and in solid tissues. Analysis of mRNA expression cannot be associated to cell numbers, since it determines an overall amount of a certain transcript in a sample. Flow cytometric analysis is problematic for solid tissues, since dissociation into a single cell suspension is required for analysis and IHC is at best semiquantitative.

It was proposed that tumor growth is facilitated by elevated levels of CD4+CD25+ assumed Treg cells, since in hepatocellular, gastric and esophageal cancers increased numbers of these cells have been observed. Reports have also associated lower levels of intratumoral CD4+CD25+FOXP3+ cells with better outcome in ovarian cancer and lower risk of recurrence in stage I non-small cell lung cancer patients.

The persuasive thought that the number of tumor infiltrating Treg inversely correlates with patient prognosis is supported by data showing abrogation of immunological unresponsiveness in a murine tumor model upon removal of CD4+CD25+ T-cells.

However, other studies do not confirm a prognostic value, or even portend a favorable prognosis for patients with an increased level of Treg [Tzankov, A., et al., Correlation of high numbers of intratumoral FOXP3+ regulatory T cells with improved survival in germinal center-like diffuse large B-cell lymphoma, follicular lymphoma and classical Hodgkin's lymphoma. Haematologica, 2008. 93(2): p. 193-200]. Since all known Treg-expression markers are also displayed by subsets of activated effector T-cells, accurate quantification of Treg is blurred and it remains unclear if elevated CD25+CD4+ cell counts in tumors reflect, at least in part, transiently FOXP3 expressing CD25+ effector T-cells rather than natural Treg. This ambiguity may be a reason for the conflicting results reported on the influence of Treg in disease outcome.

Thus, while the measurement and determination of CD4 and CD8 cells is generally easy and is usually achieved through analyzing the expression of said antigens on the cellular surface, clinically, it remains challenging to determine these cell types, since for the commonly used FACS analysis the cell samples need to be freshly isolated or immediately fixated in order to keep the cell entities intact. Thus, the detection of T lymphocytes, while desirous, is problematic, particularly for routine applications.

Lin Zhang, et al. (in: Intratumoral T Cells, Recurrence, and Survival in Epithelial Ovarian Cancer, N Engl J Med 2003; 348:203-213) discloses the association between intratumoral T cells, recurrence and survival in epithelial ovarian cancer.

EP 2199411 discloses a method for identifying CD3/CD4 positive T lymphocytes of a mammal comprising analysing the methylation status of at least one CpG position in the genes for CD3 γ, δ, and ε. However, the use oft he method for determining survival is not disclosed. EP 2141245 discloses a method for prognosing and/or predicting the outcome of cancer, comprising identifying FoxP3-positive CD25⁺CD4⁺ regulatory T cells (Treg) of a mammal, comprising analysing the methylation status of at least one CpG position in the gene for FoxP3. EP 2141245 does not detect overall T-lymphocytes (oTL) nor does it determine the ratio between oTL and Treg.

In view of the above, it is an object of the present invention to provide an improved and in particular robust method based on DNA methylation analysis to determine cancer patient survival based on the detection of tumor-infiltrating T lymphocytes. Another object would be to provide an improved method for prognosing the outcome of a cancer treatment in a patient based on the detection of tumor-infiltrating T lymphocytes.

In a first aspect thereof, the present invention solves the above objects by providing a method for determining cancer patient survival, comprising analyzing the number and/or amount of tumor-infiltrating overall T-lymphocytes (oTLs) based on the methylation status of at least one CpG position in one or more of the genes for CD3 γ, -δ, and -ε in a tumor sample derived from said cancer patient, wherein a high number and/or amount of oTLs is indicative for a better survival and/or better progression free survival of said cancer patient in a non-breast cancer.

In contrast to the situation in many if not all other cancer samples as analyzed, the data as obtained with breast cancer samples (with the identical assay as described herein) indicated a significantly decreased progression free survival in presence of tumor-infiltrating T-lymphocytes. Thus, depending on the examined tumor entity (breast vs other cancers) an abundance of T-lymphocytes in the tumor site might thus be beneficial or harmful to progression free survival.

It appears that solid tumors highly express proangiogenic factors like VEGF and FGF in a stage-dependent manner. Therefore, the more the tumor is progressed, the higher the blood flow and the accumulation of blood cells and the outcome is inferior. In cases where the tumors have an initially high blood flow (such as in colorectal cancer) the angiogenesis does not substantially change the blood flow in the tumor versus normal or stage-dependent, this leads to the fact that the prognosis positively correlates with the number of oTLs.

In the context of the present invention, a "high number and/or amount of oTLs" shall mean a number and/or amount of oTLs which is increased when the median ratio of Treg-to-oTL between healthy tissue is compared with tumor tissue. One example is from 3-8% in healthy tissue to 18-25% in a tumor entity, or increased by a factor of at least between 2 to 10, preferably of at least 5 to 10.

The present invention is based on the surprising finding of the inventors that the identification of the CD3 gene as a specific epigenetic marker can greatly facilitate the clinical routine application of the analysis of the above situation. In detail, the concept of epigenetic immunophenotyping to overall T-lymphocytes (oTL) is employed in the context of the present invention. This tool allows immune cell quantification with at least equivalent precision to FACS analysis and it is adoptable for the analysis of blood and solid tissues.

The inventors' data further demonstrate that the intragenic CD3G and CD3D region is accessible for bisulfite conversion in CD3+ T-cells only. Based on this characteristic property a specific qPCR assay for sensitive quantification of T-lymphocytes was designed. This assay, optionally together with the FOXP3 qPCR assay, presents a suitable technical approach for quantification of oTL and Treg.

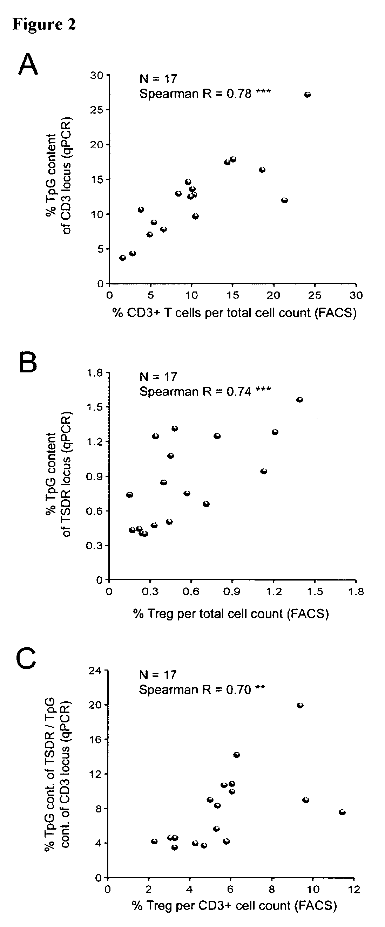

In a preferred embodiment, a region within the CpG-island of a housekeeping gene, preferably GAPDH, - which is bisulfite accessible in all cell types - can be used to determining total cell counts. Cloning the corresponding target regions of FOXP3, CD3 and GAPDH on a single plasmid (cf. EP 2 199 411 A) as equimolar quantification standard, allows for fully comparable analysis of the named cell types in any given sample. Since measurements can be performed on frozen or paraffin-embedded samples and results correlate well with cell counts obtained from FACS analysis with fresh samples (Figure 2), this technology lends itself to applications in clinical trials and routine diagnostics, where sample management remains a core challenge.

Based on this method, the inventors analyzed the frequency of Treg, oTL and their ratio in independent cohorts of healthy and tumorous ovarian, colorectal, breast and bronchial tissues with 616 partly donor-matched samples. The inventors found a shift of the median ratio of Treg-to-oTL from 3-8% in healthy tissue to 18-25% in all tumor entities. Importantly, epigenetically determined oTL-counts correlated with the outcome of colorectal and ovarian cancers. Thus, the data shows that the composition of immune cells in tumor microenvironments can be quantitatively assessed by epigenetic measurements. This composition is disturbed in solid tumors, indicating a fundamental mechanism of tumor immune evasion. In contrast to immunohistochemistry, epigenetic quantification of T-lymphocytes serves as independent clinical parameter for outcome prognosis.

In another preferred embodiment of the method according to the present invention, a demethylation of at least one CpG position to at least 90% in said sample is indicative for a CD3⁺ T-lymphocyte cell, in particular a CD3⁺ CD4⁺, and/or CD3⁺ CD8⁺ T-lymphocyte cell. Preferably, said at least one CpG position in said sample is demethylated to more than 91% and preferably more than 92% and most preferred more than 95%.

In one preferred embodiment one very good region is either the promoter or the TLSDR with e.g. the nucleotide sequence as described in, for example, EP 2 199 411 A and others, containing many CpG motifs, which display a differential methylation status when cells expressing CD3 in either CD4⁺ or CD8⁺ cells compared with all other cells, not expressing CD3 if, for example, the bisulphite sequencing method is used.

The inventors could demonstrate earlier that in CD3⁺ cells the CpG motifs are almost completely demethylated (i.e. to more than 70%, preferably 80%, preferably, more than 90% and most preferred more than 95%), whereas the same motifs are completely methylated in all CD3- cells. The differential methylation of the CpG motifs within the aforementioned region correlates with CD3 expression. Thus, determination of the methylation status of the *CD3* locus is a valuable tool to identify T-lymphocytes, such as will be required/or at least of some value for measuring T-lymphocytes in the cancers as described. The assays allows measurement of T-lymphocytes without purification or any staining procedures, and reports in solid tumors or other solid tissues the number of cells demethylated in said region, thus showing the total amount of CD3 positive tumor infiltrating T-lymphocytes.

The inventors have shown that the potential for constitutive expression of CD3 in T-lymphocytes coincides with epigenetic, i.e., DNA methylation based regulation. DNA methylation is a biologically and chemically stable epigenetic modification, resulting in long-term gene expression changes. The inventors found demethylation at the human CD3 locus to be restricted to T-lymphocytes when tested against all major peripheral blood cell types and a selection of non-blood cells, including various tumor cell lines. These data indicated that epigenetic modifications in the CD3 locus serve as valuable marker for the identification of cells with the phenotype of T-lymphocyte, regardless of the expression of the specific delta or gamma sub-chains.

It is further preferred in the context of the method according to the present invention to determine the ratio of Treg-to-oTL in said sample, based on the markers (specific for Treg vs non-Tregs, respectively), and methods as described herein.

In another preferred embodiment of the method according to the present invention, said at least one CpG position is present in the 5' region upstream from the transcription start, promoter region, intron, and/or exon/intron border within the CD3 gene or genetic region. The present invention also provides the surprising finding that in particularly preferred regions of the gene for CD3, the so-called "TLSDRs" (T lymphocyte specific demethylated regions as described in EP 2 199 411 A), the CpG motifs are almost completely demethylated (i.e. to more than 90%, preferably 91%, preferably, more than 92% and most preferred more than 95%), whereas the same motifs are completely methylated in all non T lymphocytes. Thus, these regions and the diagnostic uses thereof also provide a valuable and reliable tool for a diagnostic analysis according to the present invention.

In order to analyze the methylation status of CpG positions, any known method to analyze DNA methylation can be used. In a preferred embodiment of the method according to the present invention, the analysis of the methylation status comprises a method selected from methylation specific enzymatic digests, bisulphite sequencing, analysis selected from promoter methylation, CpG island methylation, MSP, HeavyMethyl, MethyLight, MS-SNuPE or other methods relying on a detection of amplified DNA, preferably a CD3-specific qPCR. These methods are well known to the person of skill, and can be found in the respective literature.

Preferably, the amplification involves a polymerase enzyme, a PCR or chemical amplification reaction, or other amplification methods as known to the person of skill, e.g. in the context of MSP, HeavyMethyl, Scorpion, MS-SNUPE, MethylLight, bisulfite sequencing, methyl specific restriction assays. With the amplification, the amplicon of the TLSDR or any other region in the CD3 gene or any paralog or ortholog as described in, for example, EP 2 199 411 A is produced that is a particularly preferred "tool" for performing the method(s) according to the present invention.

The person of skill will furthermore be able to select specific subsets of CpG positions in order to minimize the amount of sites to be analyzed, for example at least one of CpG position 1, 2, 3, 4, 5, 6, 7, 8, 9, and 10 of any amplicons as analyzed, for example those as described in EP 2 199 411 A, or other sequences in the CD3 locus. The positions are numerically counted from the 5'-end of the amplicon as generated and analyzed. Preferred are combinations of 4, 5, 6, or 7 positions, which are producing enough information in order to be informative in the context of the present invention.

In a preferred embodiment of the method according to the present invention, said method is suitable for routine application, for example on a DNA-chip. Based on the above information and the respective literature, the person of skill will be able to adjust the method as above to such settings.

In another preferred embodiment of the method according to the present invention, further a methylation analysis of at least one CpG position in a housekeeping gene, such as, for example, GAPDH, can be performed in order to normalize the amount and/or number of oTLs in said sample. A housekeeping gene is typically a constitutive gene that is required for the maintenance of basal cellular function, and is found in all human cells. Other suitable housekeeping genes are well known to the person of skill and, for example, described in Huggett J, Dheda K, Bustin S, Zumla A. Real-time RT-PCR normalization; strategies and considerations. Genes Immun. 2005 Jun;6(4):279-84; or Romanowski T, Markiewicz A, Bednarz N, Bielawski KP. Housekeeping genes as a reference in quantitative real-time RT-PCR Postepy Hig Med Dosw (Online). 2007 Sep 28;61:500-10.

In another preferred aspect of the method according to the present invention the methylation status of at least one CpG position in the genes for CD4⁺ and/or CD8, in particular CD8 beta, or in the genes for FOXP3, CD25, CD 127, CTLA4, GITR, CD45RA, CD103 GNGT2, CRTAM, IL2RB, and ZBTB32, or FLJ00060, FLJ38379, PPP6C, CD226, ZBTB7B, and TNFAIP8, preferably in the TSDR in the gene of FOXP3 is analyzed in analogy to what is described herein for CD3. These genes thus also allow the unambiguous identification of all CD3 positive T lymphocytes. Thus, in a preferred embodiment of the method according to the present invention, said at least one CpG position is present in the 5' region upstream from the transcription start, promoter region, intron, and/or exon/intron border within the gene(s) FOXP3, GNGT2, CRTAM, IL2RB and ZBTB32 among the CD3 positive T lymphocytes, as these markers are capable to segregate between CD8 and CD4 positive cells. This analysis is preferably performed simultaneously or subsequently to the analysis for the CD3 phenotype of the T-lymphocytes.

Equivalently, FLJ00060, FLJ38379, PPP6C, CD226, ZBTB7B and TNFAIP8 are capable of positively identifying CD4 expressing cells in whole blood and segregate between CD4 and CD8 positive CD3 positive cells.

The method according to the present invention is preferably performed on material derived from solid tumors, either primary or secondary (metastases). Further preferred is a method according to the present invention, wherein said cancer is selected from colorectal, endometrial, pancreatic, esophageal, prostate, bronchial, breast and/or ovarian cancer, and/or melanoma and/or non-small cell bronchial carcinoma.

Another preferred aspect of the method according to the present invention then further includes a prognosis based on said determination, wherein a high number and/or amount of tumor-infiltrating oTLs (optionally compared to healthy tissue or non-malignant tissue) is indicative for a better prognosis for said non-breast cancer patient. A better prognosis shall include at least one of longer overall survival, relapse-free period, progression free period and lack of metastasis for the cancer patient. In breast cancer, a high number and/or amount of tumor-infiltrating oTLs (optionally compared to healthy tissue or non-malignant tissue) is indicative for an inferior prognosis for said breast cancer patient.

In yet another preferred embodiment of the method according to the present invention, a better prognosis is associated with oTL-counts higher than the median in healthy tissues, such as, for example of 7%, or higher.

In yet another preferred embodiment of the method according to the present invention, the sample is selected from a solid tumor sample, mammalian body fluid, including human blood samples, or a tissue, organ or cell type blood sample, a sample of blood lymphocytes or a fraction thereof. Preferably, said cancer patient is a mouse, rat, monkey or human. The samples can be suitably pooled, if required.

Another preferred aspect of the method according to the present invention then relates to a method as above, further comprising measuring and/or monitoring the number and/or amount of said oTLs in response to chemical and/or biological substances that are provided to said cancer patient, for example as an anti-cancer treatment, for example a chemotherapy against colorectal, endometrial, pancreatic, esophageal, prostate, bronchial, breast and/or ovarian cancer, and/or melanoma and/or non-small cell bronchial carcinoma, preferably colon cancer or ovarian cancer.

Described is a method for treating cancer, such as colorectal, endometrial, pancreatic, esophageal, prostate, bronchial, breast and/or ovarian cancer, and/or melanoma and/or non-small cell bronchial carcinoma, in particular colon, breast, bronchial or ovarian cancer, comprising an anti-cancer treatment, for example a chemotherapy against colon cancer or ovarian cancer, measuring and/or monitoring the number and/or amount of said oTLs in response to chemical and/or biological substances that are provided using a method of the present invention as described herein, wherein a high number and/or amount of oTLs, optionally compared to an untreated cancer patient, is indicative for a treatment of said non-breast cancer.

Further described is an improved method for treating cancer, such as colorectal, endometrial, pancreatic, esophageal, prostate, bronchial, breast and/or ovarian cancer, and/or melanoma and/or non-small cell bronchial carcinoma, in particular colon, breast, bronchial or ovarian cancer, comprising measuring and/or monitoring the number and/or amount of said oTLs in response to chemical and/or biological substances that are provided to said cancer patient, for example as an anti-cancer treatment, for example a chemotherapy against colorectal, endometrial, pancreatic, esophageal, prostate, bronchial, breast and/or ovarian cancer, and/or melanoma and/or non-small cell bronchial carcinoma, I particular colon cancer, breast, bronchial or ovarian cancer, and deciding on changing, continuing or stopping said anti-cancer treatment, for example said chemotherapy against colorectal, endometrial, pancreatic, esophageal, prostate, bronchial, breast and/or ovarian cancer, and/or melanoma and/or non-small cell bronchial carcinoma, in particular colon cancer or breast, bronchial or ovarian cancer, based on changes of the number and/or amount of said oTLs in response to chemical and/or biological substances as provided to said cancer patient.

Yet another preferred aspect of the present invention then relates to the use of a kit for performing a method according to the present invention as described above, comprising materials for identifying, measuring and/or monitoring the number and/or amount of oTLs a cancer patient based on the analysis of the methylation status of CpG positions in the gene CD3 according to a method according to the present invention as described above. Preferably, said kit to be used comprises a) a bisulfite reagent, and b) materials for the methylation analysis of CpG positions in the gene CD3 according to a method according to the present invention as described above.

The data of the present invention demonstrate that the intragenic CD3G and CD3D region is accessible for bisulfite conversion in CD3+ T-cells only. Based on this characteristic property a specific qPCR assay for sensitive quantification of T-lymphocytes was designed. Together with the FOXP3 qPCR assay, this assay presents a suitable technical approach for quantification of oTL and Treg. A region within the CpG-island of the housekeeping gene GAPDH - which is bisulfite accessible in all cell types - can be used to determining total cell counts. Cloning the corresponding target regions of FOXP3, CD3 and GAPDH on a single plasmid as equimolar quantification standard, allows for fully comparable analysis of the named cell types in any given sample. Since measurements can be performed on frozen or paraffin-embedded samples and results correlate well with cell counts obtained from FACS analysis with fresh samples (Figure 2), this technology lends itself to applications in clinical trials and routine diagnostics, where sample management remains a core challenge.

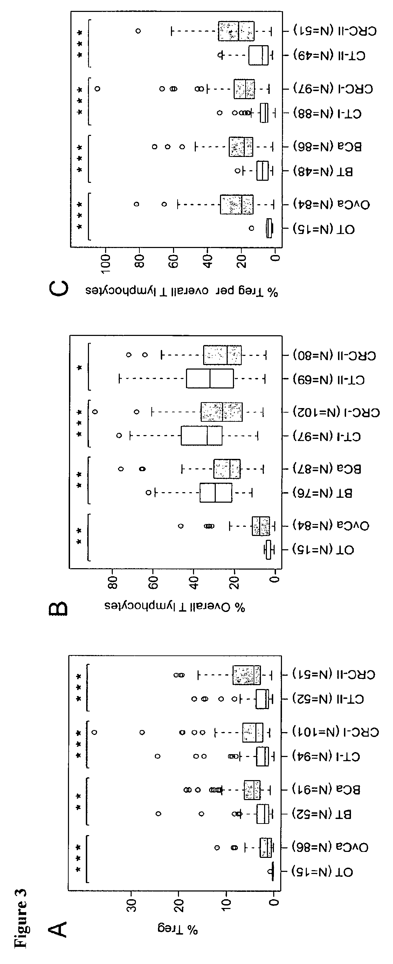

In the present invention, the technology was applied to elucidate the role of immune cells in disease-affected tissues of patients with solid ovarian, bronchial, and colorectal tumors. The data showed significantly lower infiltrates of Treg and oTL in healthy ovarian tissues when compared to other analyzed tissues. The inventors attribute this observation to tissue-specific levels of perfusion. Nonetheless, the mean ratio of Treg-to-oTL stably ranges between 3.5-7% in all healthy tissues (Figure 3C) which corresponds to the ratio measured in peripheral blood (Figure 2) and was reported elsewhere using FACS analysis. This shows that healthy immunological balance is obtained when up to one tenth of T-lymphocytes exhibit a suppressive phenotype. In all tested tumor entities, the inventors observed higher frequencies of Treg (Figure 3A). oTL-counts were reduced in lung and colorectal but were increased in ovarian tumor samples (Fig. 3B). Given that T-lymphocyte counts in healthy ovarian tissue had shown lower values than in other tissues (likely due to lower tissue perfusion), the inventors assume that the strikingly enhanced Treg-levels and notably increased oTL-level are owed to tumor-mediated increase of perfusion. In other healthy tissues, this effect is not prominently observed, as differences in perfusion of tumor and healthy tissue are less pronounced.

The ratio of Treg-to-oTL was determined in order to access the immune status excluding perfusion-associated factors. The inventors observed at least a doubling of the median level of suppressive T-cells within the total subset of tissue-infiltrating immune T-cells in tumors. Notably, this shift was observed throughout all tumor entities. The solidity of this study was further aggravated by virtual result congruence when comparing data from the two independent CRC cohorts (Figure 3).

Furthermore, the matched-pair analysis according to the present invention showed relative enrichment of Treg in 153 out of 177 (86%) patients. Exceptions to this trend were mainly observed in colorectal samples. Since histological tissue allocation is particular demanding in those samples, the inventors consider technical problems at tissue selection a feasible explanation for outliers.

CD25+FOXP3+ cells show upregulation in tumor tissues. Low numbers of CD25+CD4+FOXP3+ cells and high numbers of CD3 mRNA have been associated with better survival of cancer patients. However, the human studies were afflicted with the problem that CD25+FOXP3+ cells include activated effector T cells and tissue analysis using either mRNA, IHC or FACS analysis only provides limited accuracy of absolute and relative quantification. Using a different technical approach, here the inventors showed that a dysbalanced ratio of Treg-to-oTL infiltrates is a prominent and highly frequent observation in solid tumors. The epigenetic analyses of FOXP3 and CD3 have exclusively been associated with Treg and oTL and provide precise and fully quantitative data. The uniform dysbalance throughout various tumor entities joint with previous reports suggest that this effect is a consistent defect of the solid tumor associated immune status. The inventors assume that this dysbalance contributes to the pathological inability of the body to counter tumor establishment and likely is a prerequisite for permanent tumor establishment. Due to its descriptive nature, however, epigenetic analysis falls short of formally proving such hypothesis.

In primary human tissues, survival analyses are used as approximation to the evaluation of functional significance. Accordingly, the inventors tested the role of T-lymphocyte infiltration in tumors on patient survival. The inventors' data corroborate previous reports, which indicate that an increased number of intratumoral oTL lead to better prognosis outside of breast cancer, where the situation is opposite. This trend is observed for all three independent studies for progression free survival (PFS) and the associated overall survival (OS) analyses. Qualitative equivalence of data throughout all cohorts for both PFS and OS indicate high relevance of this observation. Accordingly, the development of an epigenetic prognostic tool for routine applications is possible. The positive effect of oTL is in line with the hypothesis that readiness of tumor-infiltrating T-lymphocytes, and thus the ratio between Treg-to-oTL directly influences the evolving tumor. This is because overall T-lymphocytes predominantly represent effector T-cells.

In summary, the inventors present evidence that epigenetic analysis of immune cells is a novel method that facilitates immunophenotyping in blood and, possibly more importantly, in solid tissues. The shown data firmly establish a prominent role of the ratio of Treg-to-oTL during tumor establishment. Despite lack of formal prove, the data doubtlessly promote the view that a dysbalance between suppressive Treg and effector T-lymphocytes coincides with tumor immune evasion and may be an intrinsic characteristic of tumor establishment. Hence, alterations of the ratio of cells of the tolerogenic-to-effector immune system may be a strong target for anti-tumor strategies. The positive effect of intratumoral T-lymphocyte counts on the prognosis of patients further supports the view that the presence and balance of immune cells is an important part of the bodies failing anti-tumor response.

The invention will now be further described based on the following examples and with reference to the accompanying figures and the sequence protocol, without being limited thereto. In the Figures,

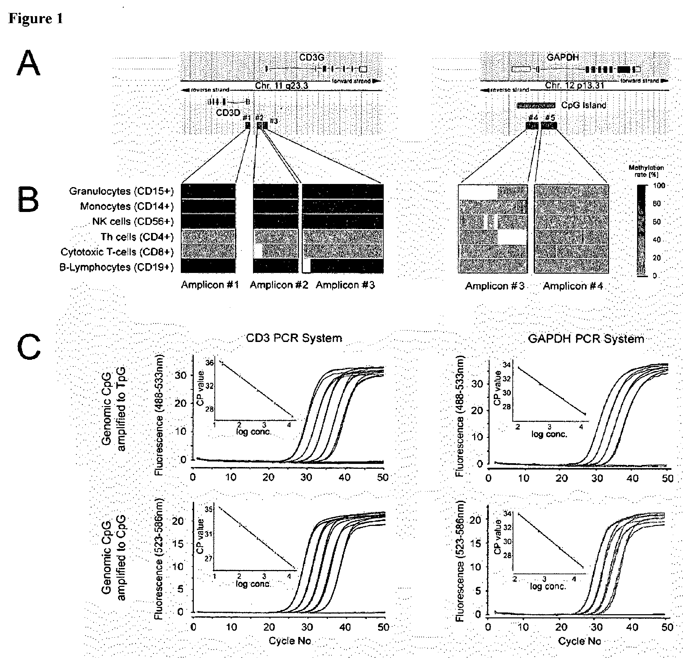

Figure 1 shows the epigenetic profiling of the CD3 and GAPDH loci. A) Genomic localization and organization of the genes. Transcripts are shown depending on their orientation above or below the chromosomal bar. Amplicons used for epigenetic analysis are indicated as red boxes. B) Matrix of the epigenetic profiling obtained from bisulfite sequencing of the amplicons shown in A. Each line represents the bisulfite-conversion status of the CpGs present in the amplicon tested on the purified cell types as indicated on the left. Each individual square represents a single genomic CpG-position. Blue squares correspond to genomic CpGs that are inaccessible to bisulfite-conversion and are detected as CpG (CpG-variant). Yellow corresponds to CpGs that are accessible to bisulfite-conversion and are sequenced as TpGs (TpG-variant). The grey-shade code is shown on the right hand side. C) Amplification profiles of bisulfite-conversion specific qPCR assays. In the upper panels the PCR system specific for the TpG-variant is tested on serial dilutions of 12500, 2500, 500, 100 and 20 plasmid copies representing the TpG (light grey) and CpG template variants (dark grey). In the lower panels, the same experiment is performed using the PCR system specific for the CpG-variant. Linearity of all PCR systems is shown inside of each graph by plotting measured CP values over the log concentration of template used.
Figure 2 shows CD3+ and FOXP3+ cell counting using epigenetic qPCR and FACS. Peripheral blood was collected from 17 donors and cell numbers were measured within 4-8 h by FACS (X-axis) and within one year by qPCR analysis (Y-axis). A) Cell numbers counted by FACS describe the percentage of CD3+ cells per all nucleated cells. qPCR analysis was performed applying the CD3-specific qPCR system. B) Cell numbers counted by FACS describe the percentage of CD4+CD25+CD127- per all nucleated cells. qPCR analysis was performed using the FOXP3-specific qPCR system. C) Cell numbers counted by FACS describe the percentage of CD4+CD25+CD127- within CD3+ cells. R indicates the Spearman rank correlation coefficient for each FACS analysis compared to the epigenetic measurement. Statistical significance is indicated as follows: 2 asterisks (**) p<0.01 and 3 asterisks (***) p<0.001.
Figure 3 shows the frequency of tissue infiltrating lymphocytes in healthy and cancerous tissues. Boxplots showing the percentage of A) Treg, B) CD3+ T-cells, C) Treg within CD3+ T-cells in healthy and cancerous tissues. OT, BT and CT display healthy ovarian, bronchial and colorectal tissue, respectively. OvCa, BCa and CRC indicate cancerous ovarian, bronchial and colorectal tissues. N indicates the number of patients included in each plot. The box depicts the middle 50% of the distribution. The line in the box represents the median of the distribution while whiskers extend to covering 95% of all measured data. Outliers from this distribution are indicated by circles. The statistical significance is indicated as follows: 1 asterisk (*) describes p<0.05, 2 asterisks (**) p<0.01 and 3 asterisks (***) p<0.001.

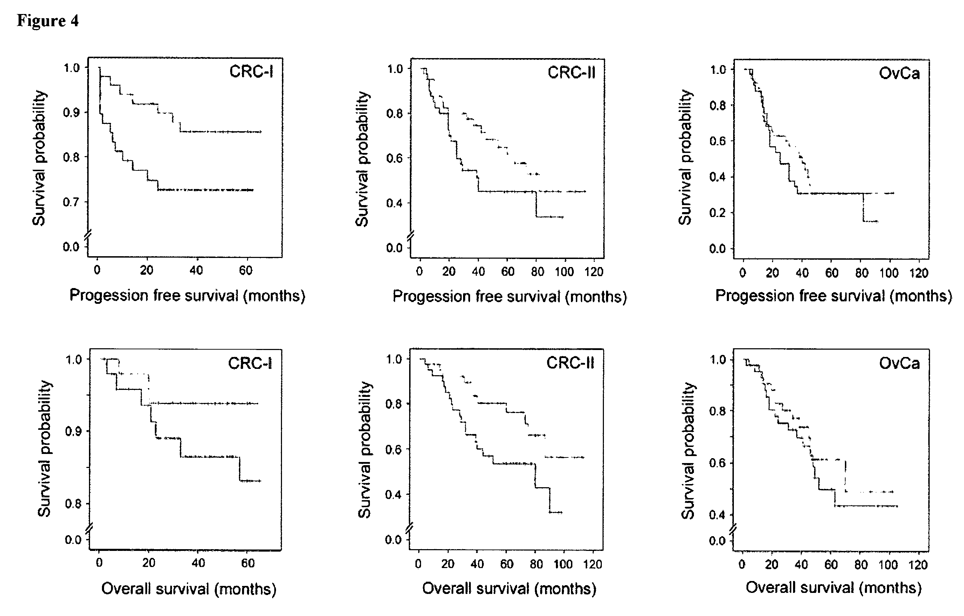

Figure 4 shows the cumulative survival of patients with colorectal and ovarian cancer. Samples from each tumor entity were divided into two groups at their median value of tumor-infiltrating overall T-lymphocytes (oTL) as measured by CD3-specific qPCR. Levels above the median (high oTL numbers) are represented by a light grey (upper line) and levels below the median (low oTL numbers) by a darker grey line (lower line). A) Progression free survival (PFS). In the left (CRC-I; median=26.1%; Nhigh=7; Nlow=12) and middle (CRC-II; median=23.9%; Nhigh=17; Nlow=22) panel colorectal cancer samples are shown. The right panel shows ovarian cancer samples (OvCa; median=7.76%; Nhigh=23; Nlow=23). B) Overall survival (OS). The left panel shows the CRC-I cohort (Nhigh=3; Nlow=7), middle panel shows the CRC-II cohort (nigh=11; Nlow=19) and the right panel shows the OvCa cohort (Nhigh=14; Nlow=18).

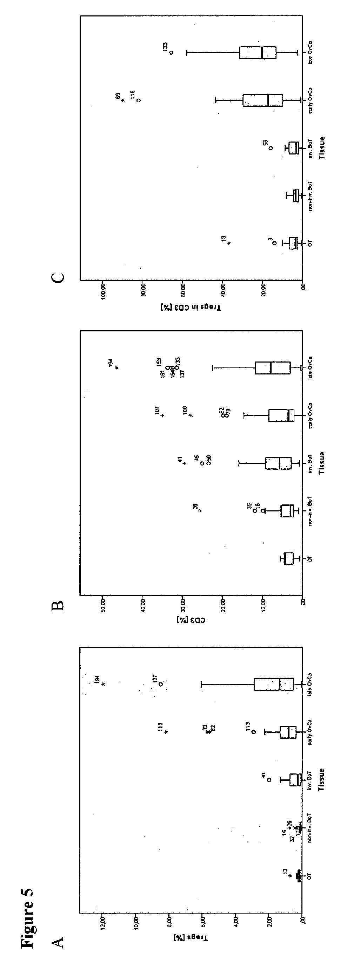

Figure 5 shows A) Tregs in ovarian tissues, B) overall T-lymphocytes in ovarian tissues, and C) the ratio of Treg in CD3 in ovarian tissues according to example 2.

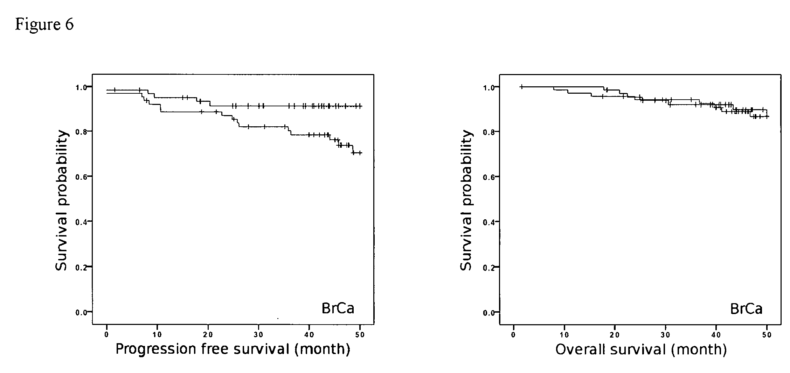

Figure 6 shows the cumulative survival of breast cancer patients. Samples were divided into two groups at the median value (15.7%) of tumor-infiltrating overall T-lymphocytes (oTL) as measured by CD3-specific qPCR. Levels above median (high oTL frequencies) are shown green; levels below median (low oTL frequencies) are blue. In the left part the progression free survival (Nhigh = 14; Nlow = 4), in the right the overall survival (Nhigh = 8; Nlow = 7) is shown. The number of samples was N_overall (PFS)=124, N_overall (OS)=135.

### EXAMPLES

### Example 1

The inventors determined the epigenetic pattern of the intergenic CD3G/CD3D region as specific marker for the identification of oTL. The inventors developed a highly sensitive and quantitative real time PCR based assay, and also provide a general reference system for total cell counting by establishing a similar epigenetic assay based on a CpG island in the regulatory region of the glycerinaldehyd-3-phosphate-dehydrogenase (GAPDH) gene. Applying those epigenetic assay systems, the inventors analyzed tissue infiltrating Treg and oTL in healthy and tumorous tissue of bronchial, colorectal and ovarian origin.

The data clearly shows that the frequency of oTL infiltration in tumors correlates with patient prognosis and that the ratio of Treg-to-oTL is dysbalanced in tumors and may be fundamental to tumor establishment.

### Cells and tissues

FFPE samples were retrieved from the archives of the Institute of Pathology, Charité Berlin, Campus Benjamin Franklin. Tissue microarray (TMA) of colorectal or bronchial carcinoma specimens with the corresponding normal parenchyma were constructed using cores of 1 mm in diameter. Fresh ovarian tissue and blood was retrieved from tumor bank ovarian cancer, Charité Berlin, Campus Virchow.

### Isolation of genomic DNA and bisulfite conversion

For purification of genomic DNA the DNeasy Blood and Tissue Kit (Qiagen) was used. Genomic DNA from FFPE samples was isolated using QIAampDNA FFPE TissueKit (Qiagen). Paraffin blocks were trimmed to remove excess of paraffin and tissue section thickness was adjusted to 10 µm. Each reaction was carried out using 10 tissue sections. Bisulfite-conversion was performed using EpiTect BisulfiteKit (Qiagen). Reactions were carried out using 0.5-1 µg genomic DNA.

### Oligonucleotides

Oligonucleotides (forward (fp) and reverse (rp) primers and probes (p)) are indicated by chromosomal positions relative to the assembly of the human genome GRCh37 (e!Ensemble release 56; Sep.09).

Bisulfite-sequencing oligonucleotides:
a) intergenic CD3G(ENSG00000160654)/CD3D(ENSG00000167286) region:
   Amplicon-No.1, fp:11:118213200-21:1, rp:11:118213616-37:1;
   No.2, fp:1 1:118214271-92:1, rp:1 1:118214685-705:1;
   No.3, fp:11:118214702-23:1, rp:11:118215151-73:1;
b) GAPDH (ENSG00000111640)
   CpG island: No.3, fp:12:6644119-35:1, rp:12:6644635-56:1;
   No.4, fp: 12:6643586-604:1, rp: 12:6643990-4011:1.

RT-PCR Oligonucleotides:
a) FOXP3 (ENSG00000049768) TSDR: CpG-specific : fp:X:49117219-46:1, rp:X:49117283-307:1, p:X:49117256-73:1; TpG-specific: 7 fp:X:49117219-46:1, rp:X:49117283-307:1, p:X:49117256-78:1.
b) CD3: CpG-specific: fp:11:118213633-53:1, rp:1 1:118213686-707:1, p: 11:118213670-87:1; TpG-specific: fp:11:118213632-53:1, rp:11:118213686-709:1, p:11:118213664-90:1. c) GAPDH: TpG specific: fp:12:6644378-99:1, rp:12:6644456-76:1, p:12:6644429-57:1.

### Bisulphite sequencing

PCR was performed in 25ul containing 7ng DNA, 1xPCR Buffer, 1U Taq DNA polymerase (Qiagen), 200uM dNTP, 12.5 pmol primer. Thermocycling conditions: 1 x 95°C, 15min, 40 x (95°C, 1 min; 55°C, 45 s; 72°C, 1 min); 1 x 72°C, 10 min. PCR products were purified using ExoSAP-IT (USB Corp.) and sequenced with amplification primers and BigDye Terminator v1.1 chemistry (Applied Biosystems). Products were Ethanol-precipitated, dissolved in 1M betain and subjected to capillary electrophoresis on ABI 3100 genetic analyzer. ABI files were interpreted using ESME (Lewin, J., et al., Quantitative DNA methylation analysis based on four-dye trace data from direct sequencing of PCR amplificates. Bioinformatics, 2004. 20(17): p. 3005-12.).

### Real-Time PCR (qPCR)

qPCR was performed using Roche LightCycler 480 Probes Master chemistry or Epitect-MSP (Qiagen) in 20 µl containing 30 pmol of each primer, 5 pmol probe, 50ng λ-phage DNA (New England Biolabs) and 60 ng DNA-template or the corresponding amount of plasmid standard. Each sample was analyzed in triplicates. For all assays cycling conditions were: 1 x 95°C, 10 min; 50 x (95°C, 15 s; 61°C, 1 min). CP ("crossing point") was computed by second derivative maximum method (LC480 software). Copy numbers were calculated from calibration curves (using serial dilutions of plasmid-based standards) by linear regression.

### Plasmid standard

Bisulfite-converted methylated, and bisulfite-converted demethylated target regions for the various real-time PCR based assays were designed *in silico*, synthesized (Genscript Inc.) and fragments were inserted into plasmid pUC57. Recombinant plasmids were linearized and serially diluted in 10ng/µl of λ-phage DNA (New England Biolabs) to obtain standards for real-time PCR based assays with final concentrations of 12500, 2500, 500, 100 and 20 template copies per reaction.

### Cell sorting of major peripheral blood leukocyte populations

Peripheral blood samples were obtained from healthy donors in accordance with local ethical committee approval. Fractionation of blood samples into different leukocyte populations such as granulocytes (CD15+), monocytes (CD14+), CD4+ T-cells (CD3+CD4+), Treg (CD4+CD25highCD45RA-), B-cells (CD19+), NK-cells (CD56+, CD56bright, CD56dim), naive CD8+T-cells (CD3+CD8+CD45RA+CD127+) and memory CD8+T-cells (CD3+CD8+CD45RA-CD127+/-) was performed as described previously [Baron, U., et al., DNA demethylation in the human FOXP3 locus discriminates regulatory T cells from activated FOXP3(+) conventional T cells. Eur J Immunol, 2007. 37(9): p. 2378-89]. Purities of sorted cells were >97% as determined by flow cytometry and viabilities were always >99%.

### Statistical Analysis

Template copy numbers were estimated from calibration curves by linear regression on crossing points from the second-derivative maximum method [Rasmussen, R., Quantification on the LightCycler, in Rapid cycle real-time PCR, methods and applications. W.t.C. Meuer S, Nakagawar a K, Editor. 2001, Springer Press: Heidelberg. p. 21-34]. The median was used to aggregate triplicate measurements of tested samples. The proportion of gene-specific DNA was computed as ratio of the gene specifically TpG-variant DNA and either the sum of the TpG and CpG-variants of this gene or the number of GAPDH TpG-variant copies. Cumulative survival was calculated by the Kaplan-Meier method [Kaplan, E.G., Civic Hospital; a report of its first year of operation. J Am Podiatry Assoc, 1958. 48(3): p. 112-3]. For univariate comparison, statistical significance was assessed using the Cox-Mantel test [Mantel, N., Evaluation of survival data and two new rank order statistics arising in its consideration. Cancer Chemother Rep, 1966. 50(3): p. 163-70]. For correlation analysis, Spearman rank correlation statistics were used. Median differences were tested with Wilcoxon rank sum (for OT and OvCa cohort) or Wilcoxon signed rank tests (for BT, BCa and both CT and CRC cohorts) depending on the sampling method. All p-values are two-sided. For analysis, statistics software SAS 9.2 (TS2M2) (SAS Institute Inc., Cary NC, USA) was employed.

### Cell type specific gene regions susceptible for complete bisulfite conversion

The inventors tested bisulfite-convertibility of CpG dinucleotides in the intergenic control region of the CD3D and CD3G genes and the CpG-island in the GAPDH gene by means of bisulfite sequencing (Figure 1A). The inventors found that all cytosines of the analyzed CD3 region were completely converted in naive CD4+ (including natural Treg) and CD8+ T-lymphocytes resulting in the TpG-variant only (Figure 1B). Bisulfite conversion in other cell types, including granulocytes, monocytes, B-lymphocytes and NK cells resulted in the CpG-variant in this gene region. The inventors exclusively found the TpG-variant of GAPDH in all tested cell types (Figure 1B) and confirmed this in various complex tissues. Based on these data, the inventors designed quantitative real time PCR assays (qPCR) for the analyzed loci of CD3 and GAPDH. For each region, the inventors developed one qPCR system that exclusively recognizes the TpG template, and one qPCR system that is specific for the CpG template, including a variant specific fluorescence labeled detection probe for each assay (Figure 1C). In order to provide a copy number quantification standard, the inventors constructed plasmid systems for both loci that correspond to the TpG- and CpG-variants. The inventors showed linearity of amplification over three orders of magnitude (amplification efficiency ranged between 1.95 and 2) and the inventors did not detect cross-reactivity of each TpG- and CpG-variant specific PCR system with the mutually opposite template variant (Figure 1C).

### Characterization of the CD3- and GAPDH-specific epigenetic qPCR assays

CD3 and GAPDH qPCRs were analyzed on separated blood cell fractions purified according to [Baron, U., et al., DNA demethylation in the human FOXP3 locus discriminates regulatory T cells from activated FOXP3(+) conventional T cells. Eur J Immunol, 2007. 37(9): p. 2378-89]. Using serial dilutions of plasmids containing the equivalent of bisulfite converted TpG or CpG DNA target regions as standard, the inventors determined DNA copy numbers. The ratio of TpG/(TpG+CpG) copies was calculated for each gene region (Table 1). The results indicate that CD8+ and CD4+ T-cells contain above 99% TpG-variant for the CD3 locus, while CD19+ B-cells, CD15+ granulocytes, CD14+ monocytes and CD3-CD56+ natural killer cells consist practically exclusively (>99%) of the CpG-variant. Targeting the GAPDH locus showed that amplification of the CpG-variant failed, whereas the TpG-variant was amplified efficiently in all cell types (Table 1). Hence, quantitative analysis of GAPDH demethylation provides a cell type independent marker to detect the total DNA copy number of all cells in a given sample. In order to further demonstrate technical accuracy of both qPCR assays, we performed DNA spiking experiments. For this, the inventors selected FACS purified regulatory T-cells, whose bisulfite-converted DNA consist of >99.5% of the TpG-variant and granulocytes, which consist to >99.7% of the CpG-variant in both, CD3 and FOXP3 loci.

Using plasmids containing sequences identical to the TpG- and CpG-variants of the regions in CD3, and as positive control, FOXP3 TSDR, as well as the TpG-variant of GAPDH for normalization, the inventors quantified the relative amount of CD3 and FOXP3 TpG-variants compared to the overall cell count. For this, the inventors artificially spiked 40, 20, 10, 5, 3, 2 and 1% Treg DNA into a background of granulocyte DNA. Using the GAPDH and the specific TpG-variants of the DNA systems for relative quantification, the inventors found strict correlation between the theoretically assumed values for the spiked samples and CD3 and FOXP3 qPCR measurements (Pearson R= 0.998 for both FOXP3 and CD3).

### Analysis of Treg and overall T-lymphocytes in whole blood samples

The epigenetic markers for Treg and overall T-lymphocytes (oTL) were tested on 17 whole blood samples and results were compared to data obtained by flow cytometric analysis using the lineage specific surface molecules CD4 and CD25 for Treg and CD3 for oTL measured on the same 17 samples. The proportion of CD3+ cells as determined by FACS analysis strictly correlates to the proportion of the TpG-variant as determined by CD3 qPCR (Spearman-R = 0.78; p = 2.0E-4) (Figure 2A). Similarly, comparison of the proportion of CD4+CD25+CD127-cells obtained from FACS measurement with TpG DNA found for the FOXP3 TSDR locus showed a strong correlation (Spearman-R = 0.74, p = 7.0E-4). The measurements of the FOXP3 to CD3 ratio by either FACS or epigenetic analysis were also strongly correlated (Spearman-R = 0.7, p = 1.8E-3). For comparison Spearman rank correlation was used since goodness-of-fit testing according to Kolomov-Smirnov revealed significant deviations from normal distributions for FACS analysis, while accepting normal distribution for epigenetic measurements. This finding suggests higher frequencies of outliers for FACS analysis. Since Treg are a subgroup of the oTLs, we also investigated the interdependence of these two cell populations. The inventors observed that - in whole blood - the oTL and Treg counts correlate with each other with a noticeable Spearman correlation coefficient (R = 0.47, p = 3.8E-8).

### qPCR analysis of FOXP3 TSDR and CD3 in solid healthy and tumor tissues

To provide a fully quantitative evaluation of tissue-infiltrating FOXP3+ regulatory T-cells (Treg) and overall CD3+ T-lymphocytes (oTL) in solid tissues, the inventors compared DNA isolated from healthy and tumorous tissue of ovarian, bronchial and colorectal origin (Figure 3A-C). The median amount of infiltrating immune cells in healthy ovarian tissue (OT) is at 0.12% and 4.27% for Treg and oTL, respectively. For pathologically confirmed ovarian cancer samples (OvCa), the inventors observed a median of 1.28% Treg and 7.76% oTL. According to the nonparametric Wilcoxon rank sum test for independent samples, the elevation of both parameters in the tumors compared to healthy tissue (OT) is statistically highly significant (pTreg = 2.34E-11; poTL = 0.0066; Figure 3A-B). In healthy bronchial tissue (BT) 2.0% Treg and 29.6% oTL were observed. When testing adjacent tumor lesions (BCa), the inventors observed 4.2% Treg and 22.3% oTL. Both higher Treg counts and lower oTL counts in tumor compared to healthy tissue are statistically significant according to the Wilcoxon signed rank test for paired samples (p = 0.0024 and p = 0.0015; Figure 3A-B). The same analysis was conducted with two independent colorectal cancer cohorts comparing healthy, tumor-adjacent (CT) with tumor tissue (CRC). In the first cohort, the inventors observed median Treg and oTL counts of 1.9% and 33.5% in healthy samples (CT-I), respectively and 3.8% Treg and 26.1% oTL in tumor tissue (CRC-I), respectively (Figure 3A-B). Wilcoxon signed rank test indicates that the higher proportion of Treg (p=3.80E-4; Figure 3A) and reduced proportion of oTL (p=1.0E-4; Figure 3B) in the tumor is statistically significant. All statistical analyses remain significant after correction for multiple testing. Data from the second CRC cohort confirmed this observation showing 1.8% Treg and 32.3% of CD3 cells in healthy tissue (CT-II), contrasted by 4.3% Treg and 23.9% oTL in tumor samples (CRC-II) (p=1.0E-4 and p=0.0296) for Treg and oTL, respectively).

When correcting for multiple testing the differences between healthy and tumorous Treg and oTLs in the CRC-II cohort, the oTL value formally drops below statistical significance (p=0.1), but in the inventors' view the relevance of the trend remains undisputed.

For all four cohorts, the inventors analyzed the ratio of Treg-to-oTL (Figure 3C). In healthy tissues, the median relative Treg amount within the oTL ranged between 3-8 % (medianOT: 3.4%, medianBT: 7.6%, medianCT-I: 5.9%, medianCT-II: 7.8%). In tumor entities, this ratio is shifted towards an increased proportion of Tregs (medianOvCa:19.7%, medianBCa:18.3%, medianCRCI:17.5%, medianCRC-II:21.6%). All changes are statistically highly significant (pOvCavs.OT=8.06E-07, pBCavs.BT =1.59E-14, pCRC-Ivs.CT 3.05E-13, pCRC-IIvs.CT-II p=5.10E-7). Since for bronchial and colorectal patient cohorts donor-matched pairs of tumor and adjacent healthy tissue were used, the inventors directly compared Treg-to-oTL counts in donor-matched analysis, excluding effects from donor-to-donor variations. The inventors found that 93.5% (43 out of 46) of the pairs show an increase of the Treg-to-oTL ratio in bronchial tumor (BCa) compared to healthy adjacent tissue (BT). In the CRC-I cohort the inventors found an equivalent increase in 81% (39 of 48) of cases and in CRC-II cohort this increase is observed in 86% (71 of 83) of the pairs.

The inventors also investigated the interdependence of Treg and oTL. Similar to the correlation found in whole blood samples, the inventors observed a noticeable correlation between Treg and oTL infiltration in healthy bronchial tissue (Spearman-R=0.68, p=8.6E-08) and colon tissue (RCT-I=0.55, p=4.4E-5, RCT-II=0.54, p=2.1E-8). The same analysis with a limited number of ovarian tissue samples (N=13) did not show significant correlation (R=0.15, p=0.6). When testing this interdependence in diseased tissues the inventors observed strong correlation in ovarian cancer tissue (ROvCa=0.77, p=3.5E-21), and noticeable correlation in BCa and CRC (RBCa=0.48, p = 3.25E-8; RCRC-I=0.37, p=0.02; RCRC-II=0.58, p=1.2E-10).

### Correlating patient survival with intratumoral immune cell counts

For colorectal and ovarian cancer patients follow-up data were available. Hence, the inventors tested if the number of tumor-infiltrating oTLs correlated with patient prognosis (Figure 4). To detect the effect exhibited by the oTL number, the inventors split patients at the median oTL count into two groups. This split is arbitrary, but provides for balanced sampling sizes. In CRC cohort-I, 20 recurrences - reporting the endpoint of progression free survival (PFS) - and 10 deaths - endpoint of the overall survival (OS) - were reported with 77 and 90 censored data points, respectively. These event numbers were not sufficient for statistically significant survival analyses, but a trend for an improved PFS and OS was observed for patients with high numbers of oTL. Less than 10% of the patients with high and approx. 25% of patients with low oTL counts had suffered recurrence after 20 months. 5% of patients with high numbers and 15% of those with low numbers of oTL had died forty months after diagnosis (Figure 4). The inventors then used an independent colorectal cancer cohort (CRC-II) excluding stage I disease patients to confirm the observed trend. In this cohort, 80 patients had sufficient follow-up with 39 recurrences and 30 deaths reported. The median oTL-count in this cohort was at 23.9% (range: 4.9-72.1%). In univariate Kaplan-Maier analysis for PFS we found a clear trend towards better survival of patients with high oTL-counts (median 82 vs. 40 months). Median OS was not reached for patients with high oTL, and was at 80 months for low oTL-count indicating a statistically significant survival advantage (p=0.039). In this cohort, the inventors also determined the oTL number using IHC. Those data did not correlate with a better prognosis or with the data found in the epigenetic analysis. In the OvCa cohort, data from 67 patients were included in the PFS analysis with 45 recurrences reported. For OS analysis, 79 patients were included and 30 events were reported. Univariate analysis in the OvCa cohort suggests better prognosis for patients with oTL-counts higher than the median of (in this case) 7.76% (Figure 4). Median PFS was 40 months for patients with high and 25 months with low numbers of oTL. Similarly, median OS was better for patients with high oTL (70 months) compared with patients with low oTL counts (52 months).

For PFS and OS of all three cohorts, the inventors calculated Cox regression models. The effect of continuously increasing oTL is protective throughout all analyses, also after adjusting for age, grading, stage and other cohort-specific parameters. The hazard ratio for PFS is at 0.966 and 0.998 for OvCa and CRC-II, respectively and at 0.98 and 0.987 for OvCa and CRC-II for OS. For CRC-I, the small number of events disallows robust modeling, but-with tentative hazard ratios of 0.995 and 0.972 for PFS and OS, respectively - the trend for improved tumor response mediated by elevated T-lymphocyte numbers is further confirmed. Together, Cox regression analysis shows that risk for recurrence or death decreases by approx. 0.2-3.4 % for each 1% increasing oTL-content in the tumor environment.

### Example 2

This example aims at contributing to the understanding of the failing immune system during tumor development. For this, the inventors used cohorts of
a) ovarian cysts - representing benign ovarian tissue growth,
b) non-invasive borderline tumors - representing those (semi-) malignant tumors with the comparably best outcome of all malignant diseases of the ovary,
c) invasive borderline tumors - representing a somewhat more dangerous but still semi-malignant form of an ovarian tumor, as well as
d) early and late stage ovarian cancer - representing the most malignant form of ovarian tumors as found at different stages of their progression.

Using the epigenetic tests according to the present invention, the frequency and ratio of regulatory T cells and overall T cells in these different diseases of the ovaries was determined. In a cohort of 15 donors, a median of 0.12% of regulatory T cells (Tregs) in healthy (benign) ovarian tissues was found with the minimum value at 0.01% and a maximum of 0.71%. In non-invasive borderline tumors (n=23), a mean of 0.35% (min. = 0.06, max. = 1.48%) was found. In invasive borderline patients (n=24), this mean value further increased to 0.57% (min. = 0.06, max. = 3.96). In early ovarian cancer patients, a median Treg level of 0.8% (min. = 0.11, max. = 8.16%) was observed, and in late stage ovarian cancer the median Treg level is further increased to 1.32% (min. = 0.05, max. = 11.95%).

All comparisons with exception of the comparison early-to-late ovarian cancer were statistically significant when tested in a non-parametrical two-sided T-test. Next, the inventors analyzed the number of overall T-lymphocytes (oTLs). The frequency of these cells should indicate the "activity" of the immune system in the given tissue. A relatively stable oTL count was observed between the healthy ovarian tissue (4.27% n =15), non-invasive borderline tumors (2.93%, n =25), and early stage ovarian cancer (3.47%, n=40). An increase of the invasive borderline (5.71, n = 25) and the late ovarian cancer (7.94%, n = 77) compared to the forms of disease with comparably better outcome (i.e., non-invasive borderline, ovarian tissue and early stage ovarian cancer) was observed.

Finally, the ratio between Treg and oTLs in the different tumor entities was determined, based on the data obtained in the experiments described above. This is the most significant of all approaches, since the activity of regulatory T cells is targeted at effector T cells and the activity of the latter is determined not primarily by their abundance but by their ability to attack the tumor. This ability is severely impaired by the presence of suppressive Tregs. The inventors observed that in non-malignant ovarian tissue this ratio is at approximately 3.5 %. This value corresponds to the ratio in blood and other healthy tissues. This ratio is increased in the semi-malignant non-invasive (9.58%) and invasive borderline (10.51%) tumors to approximately 10%. The same trend is further extended in malignant tumors, where the prevalence of Tregs in the oTL population is further increased to 17.22% in early and 20.22% in later stage tumors.

In summary, increasing malignancy and increasing Treg counts, as well as a decreasing share of oTLs, i.e., the effector T-cell compartment within the tumor microenvironment are closely associated. The inventors here provide further evidence that the prevalence of Treg and oTLs is an important determinant for the ability of a tumor to prevail and to circumvent the body's response. It is tempting to speculate that the ability of tumor cells to influence the immune cell concentration is a paramount characteristic of the malignancy profile of the tumor.

**Table 1 Data from example 2: OT = ovarian tissue; non-inv BOT = non-invasive borderline tumors, inv BOT = invasive borderline tumors, OvCa = ovarian cancer**

| **Treg** | | | | | | | |
|---|---|---|---|---|---|---|---|
| | | | | | | | |

| | | | **OT** | **non-inv. BOT** | **inv. BOT** | **early OvCa** | **late OvCa** |
|---|---|---|---|---|---|---|---|
| | | | | | | | |
| **N** | | | 15 | 23 | 24 | 36 | 78 |
| **Level (%)** | | | | | | | |
| | median | | 0,12 | 0,35 | 0,57 | 0,80 | 1,32 |
| | mean | | 0,18 | 0,41 | 0,89 | 1,28 | 2,00 |
| | min | | 0,01 | 0,06 | 0,06 | 0,00 | 0,05 |
| | max | | 0,71 | 1,48 | 3,96 | 8,16 | 11,95 |
| | S.D | | 0,17 | 0,30 | 0,99 | 1,73 | 2,08 |
| **T-Test (p-Value); 2-sided** | | | | | | | |
| | vs OT | | | 0,00477 | 0,00196 | 0,00057 | 0,00000 |
| | vs non-inv. BOT | | | | 0,03032 | 0,00544 | 0,00000 |
| | vs early OvCa | | | | | | 0,05560 |
| | vs BOT | | | | | 0,00000 | |
| | | | | | | | |
| | | | | | | | |
| **oTL** | | | | | | | |
| | | | | | | | |

| | | | **OT** | **non-inv. BOT** | **inv. BOT** | **early OvCa** | **late OvCa** |
|---|---|---|---|---|---|---|---|
| | | | | | | | |
| **N** | | | 15 | 25 | 25 | 40 | 77 |
| **Level (%)** | | | | | | | |
| | median | | 4,27 | 2,93 | 5,71 | 3,47 | 7,94 |
| | mean | | 3,53 | 4,72 | 8,13 | 6,48 | 9,71 |
| | min | | 0,60 | 0,94 | 0,74 | 0,00 | 0,38 |
| | max | | 5,51 | 25,53 | 29,43 | 34,90 | 46,43 |
| | S.D | | 1,48 | 5,15 | 7,83 | 7,53 | 9,19 |
| **T-Test (p-Value); 2-sided** | | | | | | | |
| | vs OT | | | 0,28683 | 0,00817 | 0,02265 | 0,00000 |
| | vs non-inv. BOT | | | | 0,07575 | 0,26832 | 0,00109 |
| | vs early OvCA | | | | | | 0,05560 |
| | vs BOT | | | | | 0,09416 | |
| | | | | | | | |
| | | | | | | | |
| **Treg in oTL** | | | | | | | |

| | | | **OT** | **non-inv. BOT)** | **inv. BOT** | **early OvCa** | **late OvCa** |
|---|---|---|---|---|---|---|---|
| | | | | | | | |
| **N** | | | 15 | 23 | 24 | 32 | 77 |
| **Level (%)** | | | | | | | |
| | median | | 3,38 | 9,58 | 10,51 | 17,22 | 20,22 |
| | mean | | 6,68 | 12,90 | 11,14 | 22,51 | 23,93 |
| | min | | 0,61 | 1,14 | 0,46 | 0,93 | 2,61 |
| | max | | 36,75 | 50,97 | 31,75 | 89,78 | 65,52 |
| | S.D | | 9,05 | 10,35 | 7,51 | 20,31 | 14,27 |
| **T-Test (p-Value); 2-sided** | | | | | | | |
| | vs OT | | | 0,05908 | 0,12325 | 0,00060 | 0,00000 |
| | vs non-inv. BOT | | | | 0,50814 | 0,02624 | 0,00016 |
| | vs early OvCa | | | | | | 0,05560 |
| | vs BOT | | | | | 0,00000 | |

## Claims

1. A method for determining cancer patient survival, comprising analyzing the number and/or amount of tumor-infiltrating overall T-lymphocytes (oTLs) based on the methylation status of at least one CpG position in one or more of the genes for CD3 γ, -δ, and -ε in a tumor sample derived from said cancer patient, wherein a high number and/or amount of oTLs is indicative for a better survival and/or better progression free survival in a non-breast cancer patient, and wherein a high number and/or amount of oTLs is indicative for an inferior survival and/or inferior progression free survival in a breast cancer patient.

2. The method according to claim 1, wherein a demethylation of at least one CpG position to at least 90% in said sample is indicative for a CD3⁺ T-lymphocyte cell, in particular a CD3⁺ CD4⁺, and/or CD3⁺ CD8⁺ T-lymphocyte cell.

3. The method according to claim 1 or 2, wherein the ratio of Treg-to-oTL is determined in said sample.

4. The method according to any of claims 1 to 3, wherein said at least one CpG position is present in the 5' region upstream from the transcription start, promoter region, intron, and/or exon/intron border within the CD3 genetic region.

5. The method according to any of claims 1 to 4, wherein the analysis of the methylation status comprises a method selected from methylation specific enzymatic digests, bisulphite sequencing, analysis selected from promoter methylation, CpG island methylation, MSP, HeavyMethyl, MethyLight, Ms-SNuPE or other methods relying on a detection of amplified DNA, preferably a CD3-specific qPCR.

6. The method according to any of claims 1 to 5, further comprising a methylation analysis of at least one CpG position in a housekeeping gene, such as, for example, GAPDH.

7. The method according to any of claims 1 to 6, further comprising a methylation analysis of at least one CpG position in the genes for CD4⁺ and/or CD8, in particular CD8 beta, or in the genes for FOXP3, CD25, CD127, CTLA4, GITR, CD45RA, CD103 GNGT2, CRTAM, IL2RB, and ZBTB32, or FLJ00060, FLJ38379, PPP6C, CD226, ZBTB7B, and TNFAIPB, preferably in the TSDR in the gene of FOXP3.

8. The method according to any of claims 1 to 7, wherein said cancer is selected from colorectal, endometrial, pancreatic, esophageal, prostate, bronchial, breast and/or ovarian cancer, and/or melanoma and/or non-small cell bronchial carcinoma, in particular breast and/or ovarian cancer and/or melanoma.

9. The method according to any of claims 1 to 8, further comprising a prognosis based on said determination, wherein a high number and/or amount of tumor-infiltrating oTLs is indicative for a better prognosis for said non-breast cancer patient, wherein a high number and/or amount of oTLs is indicative for a inferior survival and/or inferior progression free survival in a breast cancer patient.

10. The method according to claim 9, wherein a better prognosis is associated with oTL-counts higher than the median, such as, for example of 7%.

11. The method according to any of claims 1 to 10, wherein said sample is selected from a solid tumor sample, mammalian body fluid, including human blood samples, or a tissue, organ or cell type blood sample, a sample of blood lymphocytes or a fraction thereof.

12. The method according to any of claims 1 to 11, wherein said cancer patient is a mouse, rat, monkey or human.

13. The method according to any of claims 1 to 12, further comprising measuring and/or monitoring the number and/or amount of said oTLs in response to chemical and/or biological substances that are provided to said cancer patient.

14. Use of a kit comprising materials for identifying, measuring and/or monitoring the number and/or amount of oTLs a cancer patient based on the analysis of the methylation status of CpG positions in the gene CD3 according to a method according to any of claims 1 to 13.

## Patentansprüche

1. Verfahren zur Bestimmung des Überlebens von Krebspatienten, umfassend ein Analysieren der Zahl und/oder Menge an Tumor-infiltrierenden Gesamt-T-Lymphozyten (oTL) basierend auf dem Methylierungsstatus von mindestens einer CpG Position in einem oder mehrerer der Gene für CD3 γ, -δ und -ε in einer Tumorprobe, die von dem Krebspatienten abgeleitet ist, wobei eine hohe Zahl und/oder Menge an oTL ein besseres Überleben und/oder besseres progressionsfreies Überleben in einer nicht-Brustkrebspatientin anzeigt, und wobei eine hohe Zahl und/oder Menge an oTL ein schlechteres Überleben und/oder schlechteres progressionsfreies Überleben in einer Brustkrebspatientin anzeigt.

2. Verfahren nach Anspruch 1, wobei eine Demethylierung von mindestens einer CpG Position zu mindestens 90% in der Probe eine CD3⁺ T-Lymphozytenzelle anzeigt, insbesondere eine CD3⁺ CD4⁺- und/oder CD3⁺ CD8⁺ T-Lymphozytenzelle.

3. Verfahren nach Anspruch 1 oder 2, wobei das Verhältnis von Treg-zu-oTL in der Probe bestimmt wird.

4. Verfahren nach einem der Ansprüche 1 bis 3, wobei die mindestens eine CpG Position in der 5' Region stromaufwärts vom Transkriptionsstart, der Promotorregion, Intron, und/oder Exon-/Introngrenze innerhalb der CD3 genetischen Region vorhanden ist.

5. Verfahren nach einem der Ansprüche 1 bis 4, wobei die Analyse des Methylierungsstatus ein Verfahren ausgewählt aus Methylierungs-spezifischen enzymatischen Verdaus, Bisulfitsequenzierung, einer Analyse ausgewählt aus Promotormethylierung, CpG Inselmethylierung, MSP, HeavyMethyl, MethyLight, Ms-SNuPE oder anderen Verfahren umfasst, die auf einem Nachweis von amplifizierter DNA, bevorzugt einer CD3-spezifischen qPCR, beruhen.

6. Verfahren nach einem der Ansprüche 1 bis 5, weiter umfassend eine Methylierungsanalyse von mindestens einer CpG Position in einem Housekeeping-Gen, wie zum Beispiel GAPDH.

7. Verfahren nach einem der Ansprüche 1 bis 6, weiter umfassend eine Methylierungsanalyse von mindestens einer CpG Position in den Genen für CD4⁺ und/oder CD8, insbesondere CD8 beta, oder in den Genen für FOXP3, CD25, CD127, CTLA4, GITR, CD45RA, CD103 GNGT2, CRTAM, IL2RB und ZBTB32, oder FLJ00060, FLJ38379, PPP6C, CD226, ZBTB7B und TNFAIP8, bevorzugt in der TSDR in dem Gen von FOXP3.

8. Verfahren nach einem der Ansprüche 1 bis 7, wobei der Krebs ausgewählt ist von kolorektalem, endometrialem, Pankreas-, Speiseröhren-, Prostata-, bronchialem, Brust-und/oder Ovarialkrebs, und/oder Melanom und/oder nicht-kleine Zelle Bronchialkarzinom, insbesondere Brust- und/oder Ovarialkrebs und/oder Melanom.

9. Verfahren nach einem der Ansprüche 1 bis 8, weiter umfassend eine Prognoses basierend auf der Bestimmung, wobei eine hohe Zahl und/oder Menge an Tumor-infiltrierenden oTLs eine bessere Prognose für die nicht-Brustkrebspatientin anzeigt, wobei eine hohe Zahl und/oder Menge an oTLs ein schlechteres Überleben und/oder schlechteres progressionfreies Überleben in einer Brustkrebspatientin anzeigt.

10. Verfahren nach Anspruch 9, wobei eine bessere Prognose mit oTL-Zahlen von höher als dem Median, wie zum Beispiel von 7%, assoziiert ist.

11. Verfahren nach einem der Ansprüche 1 bis 10, wobei die Probe ausgewählt ist aus einer festen Tumorprobe, einer Säugetier-Körperflüssigkeit, einschließlich menschlichen Blutproben, oder einer Gewebe-, Organ- oder Zelltyp-Blutprobe, einer Probe von Blut-Lymphocyten oder einer Fraktion davon.

12. Verfahren nach einem der Ansprüche 1 bis 11, wobei der Krebspatient eine Maus, Ratte, Affe oder Mensch ist.

13. Verfahren nach einem der Ansprüche 1 bis 12, weiter umfassend ein Messen und/oder Überwachen der Zahl und/oder Menge der oTLs in Antwort auf chemische und/oder biologische Substanzen, die an den Krebspatienten verabreicht werden.

14. Verwendung eines Kits umfassend Materialien zur Identifizierung, Messung und/oder Überwachung der Zahl und/oder Menge von oTLs eines Krebspatienten, basierend auf der Analyse des Methylierungsstatus von CpG Positionen in dem Gen CD3 gemäß eines Verfahrens nach einem der Ansprüche 1 bis 13.

## Revendications

1. Procédé de détermination de la survie d'un patient cancéreux, comprenant l'analyse du nombre et/ou de la quantité de lymphocytes T globaux (oTL pour overall T-lymphocytes) infiltrant la tumeur, en se basant sur l'état de méthylation d'au moins une position de CpG dans un ou plusieurs des gènes pour CD3 γ, δ et ε dans un échantillon tumoral dérivé dudit patient cancéreux, dans lequel un nombre et/ou une quantité élevé(e) des oTL indique une meilleure survie et/ou une meilleure survie sans progression chez un patient souffrant d'un cancer autre que le cancer du sein, et dans lequel un nombre et/ou une quantité élevé(e) des oTL indique une survie inférieure et/ou une survie inférieure sans progression chez un patient souffrant d'un cancer du sein.

2. Procédé selon la revendication 1, dans lequel une déméthylation d'au moins une position de CpG jusqu'à au moins 90 % dans ledit échantillon indique un lymphocyte T CD3⁺, en particulier un lymphocyte T CD3⁺ CD4⁺ et/ou CD3⁺ CD8⁺.

3. Procédé selon la revendication 1 ou 2, dans lequel le rapport des Treg sur les oTL est déterminé dans ledit échantillon.

4. Procédé selon l'une quelconque des revendications 1 à 3, dans lequel ladite au moins une position de CpG est présente dans la région 5' en amont du départ de la transcription, de la région promoteur, de l'intron, de la limite exon/intron au sein de la région génétique de CD3.

5. Procédé selon l'une quelconque des revendications 1 à 4, dans lequel l'analyse de l'état de méthylation comprend un procédé choisi parmi des digestions enzymatiques spécifiques de la méthylation, le séquençage au bisulfite, l'analyse choisie parmi la méthylation du promoteur, la méthylation des îlots de CpG, les procédés MSP, HeavyMethyl, MethyLight, Ms-SNuPE ou d'autres procédés reposant sur une détection d'ADN amplifié, de préférence une qPCR spécifique de CD3.

6. Procédé selon l'une quelconque des revendications 1 à 5, comprenant en outre une analyse de la méthylation d'au moins une position de CpG dans un gène domestique, tel que, par exemple, GAPDH.

7. Procédé selon l'une quelconque des revendications 1 à 6, comprenant en outre une analyse de la méthylation d'au moins une position de CpG dans les gènes pour les CD4⁺ et/ou CD8, en particulier CD8 bêta, ou dans les gènes pour les FOXP3, CD25, CD127, CTLA4, GITR, CD45RA, CD103, GNGT2, CRTAM, IL2RB, et ZBTB32, ou FLJ00060, FLJ38379, PPP6C, CD226, ZBTB7B, et TNFAIP8, de préférence dans la TSDR dans le gène de FOXP3.

8. Procédé selon l'une quelconque des revendications 1 à 7, dans lequel ledit cancer est choisi parmi le cancer colorectal, endométrial, pancréatique, oesophagien, de la prostate, bronchique, du sein et/ou ovarien, et/ou le mélanome et/ou le carcinome bronchique non à petites cellules, en particulier le cancer du sein et/ou ovarien et/ou le mélanome.

9. Procédé selon l'une quelconque des revendications 1 à 8, comprenant en outre un pronostic basé sur ladite détermination, dans lequel un nombre et/ou ou une quantité élevé(e) des oTL infiltrant la tumeur indique un meilleur pronostic pour ledit patient souffrant d'un cancer autre que le cancer du sein, dans lequel un nombre et/ou une quantité élevé(e) des oTL indique une survie inférieure et/ou une survie inférieure sans progression chez un patient souffrant d'un cancer du sein.

10. Procédé selon la revendication 9, dans lequel un meilleur pronostic est associé à des nombres d'oTL supérieurs à la médiane, comme, par exemple de 7 %.

11. Procédé selon l'une quelconque des revendications 1 à 10, dans lequel ledit échantillon est choisi parmi un échantillon de tumeur solide, un liquide corporel de mammifère, y compris des échantillons de sang humain, ou un échantillon de sang de type tissulaire, organique ou cellulaire, un échantillon de lymphocytes sanguins ou l'une de leurs fractions.

12. Procédé selon l'une quelconque des revendications 1 à 11, dans lequel ledit patient cancéreux est une souris, un rat, un singe ou un être humain.

13. Procédé selon l'une quelconque des revendications 1 à 12, comprenant en outre la mesure et/ou le suivi du nombre et/ou de la quantité desdits oTL en réponse à des substances chimiques et/ou biologiques qui sont fournies au dit patient cancéreux.

14. Utilisation d'un kit comprenant des matériaux pour l'identification, la mesure et/ou le suivi du nombre et/ou de la quantité des oTL chez un patient cancéreux en se basant sur l'analyse de l'état de méthylation de positions de CpG dans le gène CD3 selon un procédé selon l'une quelconque des revendications 1 à 13.
